(19) Europäisches Patentamt
European Patent Office
Office européen des brevets

(11) Veröffentlichungsnummer: **0 065 217**
**A2**

(12) # EUROPÄISCHE PATENTANMELDUNG

(21) Anmeldenummer: **82103889.0**

(22) Anmeldetag: **05.05.82**

(51) Int. Cl.³: **C 07 D 249/08**, C 07 D 249/12, A 01 N 43/64

(30) Priorität: **08.05.81 DE 3118258**

(43) Veröffentlichungstag der Anmeldung: **24.11.82 Patentblatt 82/47**

(84) Benannte Vertragsstaaten: **CH DE FR GB IT LI NL**

(71) Anmelder: **HOECHST AKTIENGESELLSCHAFT, Postfach 80 03 20, D-6230 Frankfurt/Main 80 (DE)**

(72) Erfinder: **Kübel, Börries, Dr., Kuckucksweg 14, D-6233 Kelkheim (Taunus) (DE)**

(54) **5-Halogenalkyl-1.2.4-triazole und Verfahren zu ihrer Herstellung.**

(57) 5-Halogenalkyl-1.2.4-triazole der Formel

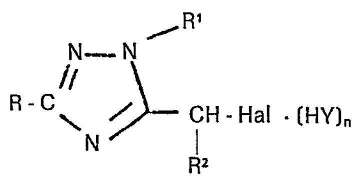

worin Hal = Cl oder Br, Y = Halogen, n = 0 oder 1, R = H, (subst.) Alkyl, Alkylthio, (subst.) Benzyl oder (subst.) Phenylethyl, $R_1$ = H, Alkyl, Cycloalkyl, Cyanethyl, Benzyl oder Alkenyl und $R_2$ = H oder $CH_3$ bedeuten, mit der Bedingung, dass im Fall R und $R^1$ = H, $R^2$ nicht H sein darf, sind Ausgangsstoffe für die Herstellung von Pestiziden.

EP 0 065 217 A2

HOECHST AKTIENGESELLSCHAFT HOE 81/F 106 0065217
Dr. AU/Rt

# 5-Halogenalkyl-1.2.4-triazole und Verfahren zu ihrer Herstellung

Gegenstand der Erfindung sind neue 5-Halogenalkyl-1.2.4-triazole der Formel I, worin

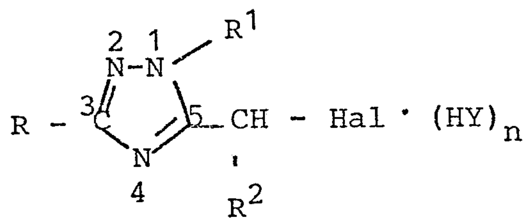
I

Hal = Chlor oder Brom,
Y = Halogen,
n = 0 oder 1
R = H, $(C_1-C_6)$-Alkyl, das durch Halogen, $(C_1-C_4)$-Alkoxy oder $(C_1-C_4)$-Alkylthio substituiert sein kann, $(C_1-C_4)$-Alkylthio, Benzyl oder Phenylethyl, die im Phenylkern durch Halogen, Methoxy oder Methyl substituiert sein können,
$R^1$ = H, $(C_1-C_6)$-Alkyl, $(C_3-C_7)$-Cycloalkyl, Cyanethyl, Benzyl oder $(C_3-C_5)$-Alkenyl und
$R^2$ = H oder Methyl bedeuten mit der Bedingung, daß im Falle R und $R^1$=H, $R^2$ nicht Wasserstoff sein darf.

Aus der Literatur sind bereits verschiedene 1.3-disubstituierte 5-Halogenalkyl-1.2.4-triazole bekannt geworden. Nach J. Chem. Soc. (C) 1970, wurden Verbindungen I mit R = Phenyl, $R^1$ = Phenyl oder Methyl und $R^2$ = H in zwei Stufen aus dem nur über Diazomethan zugänglichen 2-Phenyl-2-oxazolin-4-on erhalten. Nach Helv. Chim. Acta 61, 848 (1978) und CH-PS 574-426 können 1-Aryl-3-Carboxyl-5-chlormethyl-1.2.4-triazole aus diazotierten Arylaminen und Chloracetaminomalonester über eine Japp-Klingemann-Reaktion erhalten werden. Nach Zh. Org. Khim. 12, 2319 (1976) wurde 1.3-Diphenyl-5-chlormethyl-1.2.4-triazol aus dem Halbaminal von Dichloracetaldehyd mit Benzamid über 3 Stufen erhalten, deren eine nur 20 % Ausbeute ergab. All diesen Verfahren ist gemeinsam, daß mehrere Synthesestufen aus zum Teil nur schwierig erhältlichen Vorstufen nötig sind und daß sie ausschließlich zu Verbindungen führen, die in der 1- und/oder 3-Stellung des 1.2.4.-Triazolringes einen (gegebenenfalls substituierten) Phenylrest enthalten.

In J. Med. Chem. 19, 1057 (1976) ist eine Synthese von 3-Chlormethyl-4-aryl-1.2.4-triazolen beschrieben, deren wesentlicher Schritt eine Hydroxymethylierung in 3-Stellung eines 4-Aryl-1.2.4-triazols mittels Formaldehyd ist. 1.2.4-Triazole mit den Resten R und $R_1$ in 1.3-Position ließen sich jedoch nicht auf analoge Weise hydroxymethylieren.

Ein erfindungsgemäßes Verfahren zur Herstellung von Verbindungen der Formel I, das sich insbesondere zur Herstellung 1.3-disubstituierter Verbindungen der Formel I (d.h. $R^1$, $R \neq H$) eignet und in dieser Hinsicht den beschriebenen literaturbekannten Verfahren überlegen ist, ist dadurch gekennzeichnet, daß man Amidrazone der Formel II

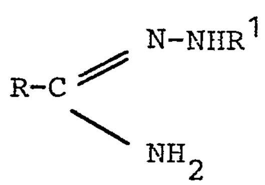

II

bzw. deren mineralsaure Salze mit α-Halogencarbonsäurehalogeniden bzw. -anhydriden der Formel III

$$R^2\text{-CH(Hal)-}\overset{O}{\overset{\|}{C}}\text{-X} \qquad \text{III}$$

umsetzt und die erhaltenen Zwischenprodukte der Formel IV dem Ringschluß mittels wasserabspaltender Mittel unterwirft.

In den Formeln III und IV bedeutet X Chlor, Brom oder den Rest $R^2$-CH(Hal)$CO_2$-. Bevorzugte Verbindungen III sind Chloressigsäureanhydrid, Chloracetylchlorid oder α-Chlorpropionylchlorid. Die Reaktion verläuft nach folgendem Schema:

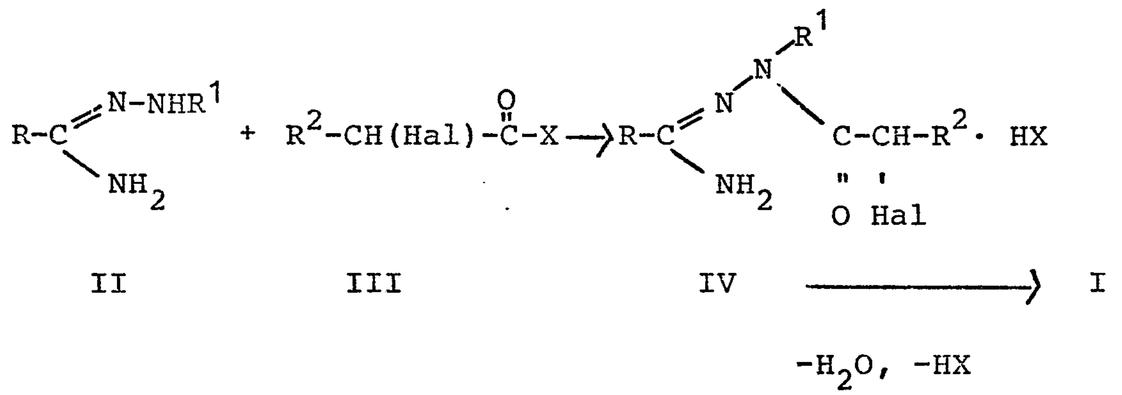


II III IV I

Als wasserabspaltende Mittel können z.B. Polyphosphorsäure, Phosphorpentoxid oder gleichwertige Mittel verwendet werden. In diesem Fall setzt man mineralsaure Salze der Verbindungen II mit III und einem Säurefänger, z.B. Triethylamin, in etwa äquivalenten Mengen bei Temperaturen von -10°C bis Raumtemperatur in aprotischen, gegen III inerten Lösungsmitteln um. Geeignete Lösungsmittel sind halogenierte Kohlenwasserstoffe wie Dichlormethan, Chloroform, 1.2-Dichlorethan, Chlorbenzol, Ketone wie Methylethylketon, Aceton oder Nitrile wie Acetonitril. Nachdem IV gebildet ist (2 - 12 h), wird entweder das Lösungsmittel im Vakuum abdestilliert und der Rückstand mit 20 bis 60 g Polyphosphorsäure oder einer entsprechenden Menge eines anderen geeigneten wasserabspaltenden Mittels je 0.1 Mol IV versetzt oder erst das wasserabspaltende Mittel zugegeben und dann das Lösungsmittel abdestilliert. Anschließend wird auf

Temperaturen oberhalb 100°C, vorzugsweise 120 bis 140°C erhitzt, wobei im Falle von X = Br oder Cl sich die entsprechende Menge Halogenwasserstoff gasförmig abspaltet. Man hält die Temperatur einige Stunden aufrecht, läßt dann abkühlen und gibt Wasser, ein nicht wassermischbares Lösungsmittel und soviel Natrium-, Kalium- oder Ammoniumhydroxid, daß ein pH-Wert von 8 - 10 erreicht wird, hinzu. Wenn alles in Lösung gegangen ist, wird die organische Phase abgetrennt und in üblicher Weise aufgearbeitet. Die Produkte können zur Reinigung destilliert oder umkristallisiert werden.

Mit Vorteil wird als wasserabspaltendes Mittel die Verbindung III selbst verwendet. Man kann in diesem Fall mit oder ohne Lösungs- bzw. Verdünnungsmittel arbeiten. Geeignete Verdünnungsmittel sind inert und haben einen Siedepunkt über 100°C. Beispiele sind Toluol, Chlorbenzol, Xylol, Mesitylen und Dichlorbenzol.

Man läßt III im Überschuß bei einer Temperatur zwischen Raum- und Rückflußtemperatur des verwendeten Lösungsmittels zu einer Suspension eines mineralsauren Salzes von II zutropfen oder gibt dieses portionsweise zu III oder dessen Lösung zu. Das Molverhältnis von II : III beträgt dabei vorzugsweise 1 : 1.9 bis 1 : 2.5. In Abhängigkeit von den Ausgangsverbindungen tritt zwischen 20° und 80°C, meistens ab 40 - 60°C, die Bildung von IV ein. Sie gibt sich im Falle von X = Chlor oder Brom an einer kräftigen Entwicklung von Halogenwasserstoffgas zu erkennen. Man hält die Reaktionstemperatur bis zum Nachlassen der Gasentwicklung bzw. bis zum Ende der exothermen Reaktion aufrecht und erhöht sie dann auf die Rückflußtemperatur des Lösungsmittels bzw. auf ca. 140°C, wobei im Falle X = Cl oder Br erneute Gasentwicklung einsetzt. Nach 2 bis 6 h ist die Reaktion

beendet. Die Aufarbeitung erfolgt ähnlich wie im vorerwähnten Fall. Häufig fallen die Verbindungen I soweit rein an, daß nicht destilliert zu werden braucht.

Die Verbindungen III sind leicht zugängliche Standardprodukte. Die Verbindungen II mit R = $CH_3$ und $R^1$ = Alkyl sind in Form ihrer Hydrochloride aus Zh. Org. Khim. 15 (1979), 2280 - 87 bekannt; andere lassen sich analog dazu durch Umsetzung von Iminoether-hydrohalogeniden oder Amidinium-Salzen mit mono-substituierten Hydrazinen in Methanol oder Ethanol herstellen.

Verbindungen der Formel I mit R, $R^2$ = H und $R^1 \neq$ H werden vorzugsweise nach einem Verfahren hergestellt, das dadurch gekennzeichnet ist, daß man Verbindungen der Formel V, worin $R^1$ = $(C_1-C_6)$-Alkyl, $(C_3-C_7)$-Cycloalkyl, Cyanoethyl, Benzyl oder $(C_3-C_5)$-Alkenyl bedeutet,

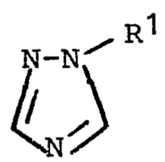

V

mit wäßrigem vorzugsweise 30 - 40 %-igem Formaldehyd im Autoklaven während einer Reaktionsdauer von ca. 20 Stunden bei einer Reaktionstemperatur im Bereich von 110°C bis 150°C zu einer Verbindung der Formel VI, worin $R^1$ die obengenannte Bedeutung besitzt, umsetzt, diese mit einem

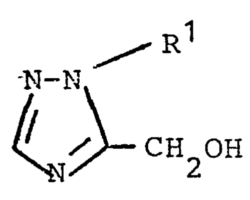

VI

anorganischen Säurehalogenid wie bspw. Thionylchlorid reagieren läßt und das erhaltene Hydrohalogenid der Formel I (n = 1) ggf. mit einer Base umsetzt. Dieses Herstellungsverfahren verläuft analog dem in J. Amer. Chem.

Soc. 77 (1955) S. 1538 beschriebenen Syntheseverfahren zur Darstellung von 3-Hydroxymethyl-triazol und 3-Chlormethyl-triazol.

Die bei der Umsetzung der Hydrohalogenide der Formel I verwendeten Basen sind bspw. wäßrige Alkalihydroxide, Alkalicarbonate oder Alkalihydrogencarbonate.

Verbindungen der Formel I mit R = $(C_1-C_4)$-Alkylthio, $R^1$ = H, $(C_1-C_6)$-Alkyl, oder $(C_3-C_7)$-Cycloalkyl und $R^2$ = H werden insbesondere nach einem Verfahren hergestellt, das dadurch gekennzeichnet ist, daß man Verbindungen der Formel VII, worin $R^1$ die obengenannte Bedeutung besitzt,

$$R^1-NH-NH-\overset{S}{\overset{\|}{C}}-NH_2 \qquad \text{(VII)}$$

mit Glykolsäure-$(C_1-C_4)$-alkylester, insbesondere Glykolsäuremethylester, in einem $(C_1-C_4)$-Alkanol in Gegenwart von Alkali-$(C_1-C_4)$-alkanolat bspw. Natriummethylat zu einer Verbindung der Formel VIII, worin

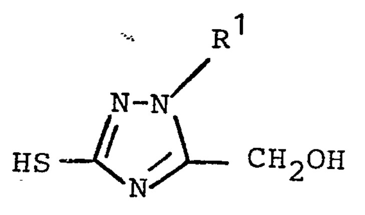

( VIII)

$R^1$ die oben genannte Bedeutung besitzt, umsetzt, diese mit einem $(C_1-C_4)$-Alkylhalogenid, insbesondere einem $(C_1-C_4)$-Alkyljodid oder -bromid an der Mercaptogruppe alkyliert und die erhaltene Verbindung der Formel IX, worin $R^1$ die obengenannte

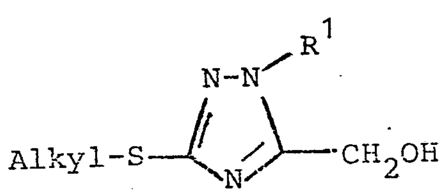

IX

Bedeutung besitzt und Alkyl einen $(C_1-C_4)$-Alkylrest bedeutet, mit einem anorganischen Säurehalogenid, wie oben beschrieben, zu einem Hydrohalogenid der Formel I (n = 1) umsetzt, das mit einer Base, wie oben beschrieben, in die entsprechende Verbindung der Formel I mit n = o überführt werden kann.

Verbindungen der Formel VII, in denen $R^1$ = $(C_2-C_6)$-Alkyl oder $(C_4-C_7)$-Cycloalkyl bedeutet, können nach einem Verfahren hergestellt werden, das dadurch gekennzeichnet ist, daß man Verbindungen der allgemeinen Formel X, worin

$$R^3{=}N{-}NH{-}\overset{\overset{S}{\|}}{C}{-}NH_2 \qquad (X)$$

$R^3$ = $(C_2-C_6)$-Alkyliden oder $(C_4-C_7)$-Cycloalkyliden bedeutet, mit einem Reduktionsmittel wie einem Borhydrid insbesondere $NaBH_4$ bei 20°C bis 60°C in einem alkoholischen Lösungsmittel wie Isopropanol hydriert.

Andere Verbindungen der Formel VII sind nach üblichen Verfahren zugänglich.

Die Hydrohalogenide der Formel I mit n = 1 können auch aus den Verbindungen der Formel I mit n = o gemäß bekannten Verfahrensweisen hergestellt werden.

Die erfindungsgemäßen Verbindungen I sind Zwischenprodukte für Pflanzenschutzmittel, insbesondere für herbizid wirksame Chloressigsäure-N-triazolylalkylanilide und für insektizide Phosphorverbindungen; z.B. lassen sich durch Umsetzung mit Alkali- und Ammoniumphosphaten bzw. -phosphonaten Phosphor- und Phosphonsäureester mit pestizider Wirkung erhalten [s. Patentanmeldung P 31 18 257.7].

Herstellungsbeispiele

Beispiel 1

5-Chlormethyl-1.3-dimethyl-1.2.4-triazol

Man löst 94,5 g (1 mol) Acetamidiniumchlorid in 500 ml Methanol, fügt 47 g (1.02 mol) Methylhydrazin zu und läßt zwei Tage bei Raumtemperatur stehen. Dann destilliert man den größten Teil des Methanols im Rotationsverdampfer ab und kristallisiert den Rückstand mit Ethylacetat. Ausbeute 111 g (90 %) N-Methylaminoacetamidiniumchlorid; weiße Kristalle mit Schmelzpunkt 110 - 120°C.

Man suspendiert 61.75 g (0.5 mol) dieser Kristalle in 400 ml Methylenchlorid und tropft unter Kühlung und Rühren erst 38 ml (0.5 mol) Chloracetylchlorid und dann während 1/2 h 70 ml (0.51 mol) Triethylamin zu. Man rührt 2 h bei Raumtemperatur, läßt noch über Nacht stehen und dampft dann im Rotationsverdampfer ein. Den Rückstand versetzt man mit 250 g Polyphosphorsäure, heizt langsam auf, wobei starke HCl-Entwicklung eintritt, und rührt dann 4 h bei 120 bis 130°C Innentemperatur. Dann gießt man auf 1 l Eiswasser, gibt 300 ml Ethylacetat und soviel Natriumhydroxid zu, daß der pH-Wert 9

wird. Man rührt, bis alles in Lösung gegangen ist, trennt die organische Phase ab und extrahiert die wässrige Phase noch dreimal mit Ethylacetat. Die vereinigten organischen Phasen werden getrocknet und das Produkt nach Abdestillieren des Lösungsmittels im Vakuum destilliert. Ausbeute 35,9 g (49 %), gelbliche Flüssigkeit vom Kp. 64°C/0.4 mbar.

Beispiel 2

1-n-Butyl-5-chlormethyl-3-methyl-1.2.4-triazol

Analog zu Beispiel 1, jedoch unter Verwendung von n-Butylhydrazin erhält man die obige Verbindung in einer Ausbeute von 49 %. Gelbliche Flüssigkeit, Kp. 90 - 92°C/0.9 mbar.

Beispiel 3

5-Chlormethyl-3-ethyl-1-methyl-1.2.4-triazol

Man löst 55.75 g (0.4 mol) Ethyl-α-iminopropyletherhydrochlorid in 200 ml Methanol, tropft unter Eiskühlung 20 g (0.43 mol) Methylhydrazin zu und läßt einen Tag bei Raumtemperatur stehen. Dann saugt man von etwas ausgefallenem Ammoniumchlorid ab und dampft das Filtrat im Vakuum ein. Man erhält 54.2 g (98 %), N-Methylaminopropionamidiniumchlorid in Form eines zähen Öls. Dieses suspendiert man in 400 ml Dichlormethan, tropft unter Rühren und Kühlen erst 30.5 ml (0.4 mol) Chloracetylchlorid und nach 1/2 h 55 ml (0.4 mol) Triethylamin zu, rührt noch 2 h unter Eiskühlung und läßt dann über Nacht bei Raumtemperatur stehen. Dann fügt man 100 g Polyphosphorsäure dazu, wodurch sich das Reaktionsgemisch unter Chlorwasserstoffentwicklung auf 35°C erwärmt. Anschließend destilliert man das Dichlormethan ab und rührt den Rückstand noch 4 h bei 120°C. Aufarbeitung wie beim Beispiel 1 ergibt 25.6 g (41 %) einer farblosen Flüssigkeit vom Kp. 74-81°C/0.8 mbar.

Beispiele 4 und 5

Analog zu den Beispielen 1 und 3 wurden erhalten:

4. 3-(4-Chlorbenzyl)-5-chlormethyl-1-methyl-1.2.4-triazol, Fp 66°C (aus Isopropylether, Ausbeute 37 %).

5. 3-Benzyl-5-chlormethyl-1-methyl-1.2.4-triazol, Gelbliches Öl, Ausbeute 41 % nach Extraktion des Rohproduktes mit Benzin und Eindampfen der Extrakte.

Beispiel 6

5-Chlormethyl-1.3-dimethyl-1.2.4-triazol

88 g (0.7 mol) des nach Beispiel 1) hergestellten N-Methylaminoacetamidiniumchlorids werden unter Rühren in 110 ml (1.44 mol) Chloracetylchlorid eingetragen. Dabei steigt die Temperatur auf 50°C und Chlorwasserstoffentwicklung setzt ein. Man rührt 15 min. bei 60°C und steigert dann die Badtemperatur auf 140 - 150°C. Nach einer Stunde ist die Chlorwasserstoffentwicklung beendet, aller Feststoff hat sich verflüssigt und die Innentemperatur hat 130°C erreicht. Nach einer weiteren Stunde wird abgekühlt. Dann fügt man unter Kühlen 4 N Natronlauge zu, bis pH 10 erreicht ist, extrahiert dreimal mit Ethylacetat, trocknet die organischen Phasen und dampft sie im Rotationsverdampfer ein. Der Rückstand wird im Vakuum destilliert. Ausbeute 59.7 g (58 %), farblose Flüssigkeit vom Kp. 70-72°C/0.5 mbar.

Beispiel 7

5-Chlormethyl-1.3-dimethyl-1.2.4-triazol

Man suspendiert 24.8 g (0.2 mol) des nach Beispiel 1 hergestellten N-Methylaminoacetamidiniumchlorids in 120 ml Chlorbenzol und tropft unter Rühren 31 ml (0.4 mol) Chloracetylchlorid zu. Dann erwärmt man auf ca. 70°C. Nachdem die Abspaltung von HCl-Gas begonnen hat, rührt man eine halbe Stunde ohne weiteres Heizen und dann noch 3 h bei Rückflußtemperatur. Man läßt abkühlen, gibt 2 N Natronlauge zu, bis ein pH-Wert von 10 erreicht ist und trennt die organische Phase ab. Die wässerige Phase wird noch dreimal mit Dichlormethan extrahiert, dann werden die vereinigten und über Magnesiumsulfat getrockneten organischen Phasen im Rotationsverdampfer eingedampft und der Rückstand im Vakuum destilliert. Ausbeute 13 g (45 %), farblose Flüssigkeit vom Kp. 72 - 74°/1 mbar.

Beispiel 8

5-Chlormethyl-1.3-dimethyl-1.2.4-triazol

31 g (0.25 mol) des nach Beispiel 1 hergestellten N-Methylaminoacetamidiniumchlorids und 85.5 g (0.5 mol) Chloressigsäureanhydrid werden miteinander vermischt und zur Schmelze erwärmt. Unter Rühren wird dann während 1/2 h auf 130° geheizt und 1 1/2 h bei dieser Temperatur gerührt. Nach dem Abkühlen erfolgt die Aufarbeitung wie in Beispiel 6 beschrieben. Ausbeute 16.4 g (42 %), Kp. 68°C/0.8 mbar.

Beispiel 9

5-Chlormethyl-1-(2-cyanethyl)-3-methyl-1.2.4-triazol

Man trägt 73 g (0.45 mol) N-(2-cyanethylamino)-acetamidiniumchlorid unter Rühren in 70 ml (0,92 mol) Chloracetylchlorid ein und erwärmt dann auf ca. 70°C. Dabei setzt Chlorwasserstoffentwicklung ein, die nach 1/2 h nachläßt. Nun steigert man die Badtemperatur auf 135°C und rührt 2 1/4 h bei dieser Temperatur. Nach dem Abkühlen wird mit NaOH alkalisch gemacht und mit Ethylacetat mehrmals extrahiert. Die vereinigten und getrockneten Extrakte behandelt man mit Aktivkohle, filtiert und engt anschließend auf ein Volumen von ca. 100 ml ein. Die dabei ausfallenden Kristalle werden abge-

0065217

saugt und verworfen. Das Filtrat wird danach im Rotationsverdampfer ganz eingedampft. Als Rückstand erhält man 34.8 g (42 %) eines braunen Öles, das nicht unzersetzt destilliert werden kann. JR-, NMR- und Massenspektrum beweisen jedoch die gewünschte Struktur.

Analog zu Beispiel 1 - 9 können folgende Verbindungen I erhalten werden:

| Beispiel | R | $R^1$ | $R^2$ | Kp. (°C/mbar) | Ausb. (%) |
|---|---|---|---|---|---|
| 10 | $CH_3$ | $C_2H_5$ | H | 68/0.65 | 53 |
| 11 | $CH_3$ | $n\text{-}C_3H_7$ | H | | |
| 12 | $CH_3$ | $i\text{-}C_3H_7$ | H | 63/0.8 | 57 |
| 13 | $CH_3$ | $n\text{-}C_4H_9$ | H | 90-92/0.9 | 55 |
| 14 | $CH_3$ | sek.-$C_4H_9$ | H | | |
| 15 | $CH_3$ | $i\text{-}C_4H_9$ | H | | |
| 16 | $CH_3$ | Cyclopentyl | H | | |
| 17 | $CH_3$ | Cyclohexyl | H | 102/0.65 | 47 |
| 18 | $CH_3$ | Cycloheptyl | H | | |
| 19 | $C_2H_5$ | $C_2H_5$ | H | | |
| 20 | $C_2H_5$ | $n\text{-}C_3H_7$ | H | | |
| 21 | $C_2H_5$ | $i\text{-}C_3H_7$ | H | | |
| 22 | $C_2H_5$ | $n\text{-}C_4H_9$ | H | | |
| 23 | $C_2H_5$ | $i\text{-}C_4H_9$ | H | | |
| 24 | $C_2H_5$ | $CNCH_2CH_2-$ | H | | |
| 25 | $C_2H_5$ | Cyclo-hexyl | H | | |
| 26 | $C_3H_7$ | $CH_3$ | H | | |
| 27 | $C_3H_7$ | $C_2H_5$ | H | | |
| 28 | $C_3H_7$ | $CNCH_2CH_2-$ | H | | |

0065217

| Beispiel | R | $R^1$ | $R^2$ | Kp. (°C/mbar) | Ausb. (%) |
|---|---|---|---|---|---|
| 29 | $i-C_3H_7$ | $CH_3$ | H | | |
| 30 | $i-C_3H_7$ | $C_2H_5$ | H | | |
| 31 | $i-C_3H_7$ | $CH_2CH_2CN$ | H | | |
| 32 | $i-C_3H_7$ | $i-C_3H_7$ | H | | |
| 33 | $n-C_6H_{13}$ | $CH_3$ | H | | |
| 34 | $n-C_6H_{13}$ | $C_2H_5$ | H | | |
| 35 | $ClCH_2C(CH_3)_2-$ | $CH_3$ | H | | |
| 36 | $Cl(CH_2)_4-$ | $CH_3$ | H | | |
| 37 | $CH_3OCH_2-$ | $CH_3$ | H | | |
| 38 | $CH_3OCH_2CH_2-$ | $CH_3$ | H | | |
| 39 | H | $CH_3$ | H | | |
| 40 | $C_6H_5CH_2-$ | $C_2H_5$ | H | | |
| 41 | $4-Cl-C_6-H_4CH_2-$ | $C_2H_5$ | H | | |
| 42 | $2-Br-C_6H_4CH_2-$ | $CH_3$ | H | | |
| 43 | $4-CH_3O-C_6H_4CH_2-$ | $CH_3$ | H | | |
| 44 | $3-CH_3O-C_6H_4CH_2-$ | $CH_3$ | H | | |
| 45 | $C_6H_5CH_2CH_2-$ | $CH_3$ | H | | |
| 46 | $C_6H_5CH(CH_3)-$ | $CH_3$ | H | | |
| 47 | $CH_3$ | $CH_3$ | $CH_3$ | 74/1.2 | 74 |
| 48 | $CH_3$ | $C_2H_5$ | $CH_3$ | | |
| 49 | $CH_3$ | $i-C_3H_7$ | $CH_3$ | | |
| 50 | $CH_3$ | $n-C_4H_9$ | $CH_3$ | | |
| 51 | $CH_3$ | $CH_2CH_2CN$ | $CH_3$ | | |
| 52 | $C_2H_5$ | $CH_3$ | $CH_3$ | | |
| 53 | $C_2H_5$ | $C_2H_5$ | $CH_3$ | | |
| 54 | $C_2H_5$ | $C_3H_7$ | $CH_3$ | | |
| 55 | $C_2H_5$ | $CH_2CH_2CN$ | $CH_3$ | | |
| 56 | $CH_3OCH_2-$ | $CH_3$ | $CH_3$ | | |
| 57 | $ClCH_2C(CH_3)_2-$ | $CH_3$ | $CH_3$ | | |
| 58 | H | $CH_3$ | $CH_3$ | | |
| 59 | $CH_3OCH_2CH_2-$ | $CH_3$ | $CH_3$ | | |
| 60 | $C_6H_5CH_2-$ | $CH_3$ | $CH_3$ | | |

| Beispiel | R | $R^1$ | $R^2$ | Kp. (°C/mbar) | Ausb. (%) |
|---|---|---|---|---|---|
| 61 | $4\text{-Cl-}C_6H_4CH_2\text{-}$ | $CH_3$ | $CH_3$ | | |
| 62 | $4\text{-}CH_3O\text{-}C_6H_4CH_2\text{-}$ | $CH_3$ | $CH_3$ | | |
| 63 | $CH_3SCH_2CH_2\text{-}$ | $CH_3$ | $CH_3$ | | |

Beispiel 64

5-Chlormethyl-1-methyl-1.2.4-triazol

a) 153 g 1-Methyl-triazol werden zusammen mit 367,5 ml wäßrigem Formaldehyd (40 %) bei 130°C unter Eigendruck (2 bis 14 bar) während 20 Stunden im Autoklaven gerührt. Nach Abdestillieren des Formalinüberschusses im Wasserstrahlvakuum werden nach zweimaligem Destillieren bei 105°C/0,2 mm 18,22 g (= 8,75 % Ausbeute) 5-Hydroxymethyl-1-methyl-1.2.4-triazol vom Fp. 79 bis 82°C erhalten.

b) Zu einer Lösung von 18,22 g 5-Hydroxymethyl-1-methyl-1.2.4-triazol in 50 ml Chloroform werden 68 ml Thionylchlorid zugetropft. Das Gemisch wird 2 Stunden bei Raumtemperatur und 2 Stunden bei Erhitzen unter Rückfluß gerührt. Man läßt über Nacht stehen, destilliert Thionylchlorid zusammen mit Chloroform im Vakuum ab und verrührt den Rückstand mit Äther. Nach Absaugen und Trocknen erhält man 25,6 g (= 94,6 % Ausbeute) 5-Chlormethyl-1-methyl-1.2.4-triazol-Hydrochlorid vom Fp. 132°C.

$^1$H-NMR-Daten *(DMSO): 3,8 (s,3H); 5,0 (s,2H); 8,1 (s,1H); 10,4 (s-breit,2H).

Durch Zugabe einer Base wie beispielsweise Kaliumcarbonat kann die Titelverbindung in Form eines Öles freigesetzt werden.

* Die $^1$H-NMR-Daten werden in ppm bezogen auf TMS als Standard angegeben.

0065217

Beispiel 65

5-Chlormethyl-1-isopropyl-1.2.4-triazol

Zu 10,6 g 5-Hydroxymethyl-1-isopropyl-1.2.4-triazol vom Kp.: 106°C/0,4 Torr (hergestellt analog der unter a) in Beispiel 64 beschriebenen Verfahrensweise. werden bei ca. 70°C 31,25 ml Thionylchlorid zugetropft und das Reaktionsgemisch 3 Stunden zum Sieden erhitzt. Nach Stehenlassen über Nacht wurde der Thionylchloridüberschuß im Vakuum abdestilliert, der Rückstand in Methylenchlorid aufgenommen und mittels 2n NaOH unter Zugabe von Eiswasser auf einen pH-Wert von 10,0 gestellt. Die wäßrige Phase wurde zweimal mit Methylenchlorid ausgeschüttelt und die vereinigten organischen Extrakte getrocknet und eingedampft. Man erhielt 7,44 g (= 62 % Ausbeute) der Titelverbindung als braunes Öl.

$^1$H-NMR-Daten ($CDCl_3$): 1,5(d,6H); 4,6(sept.)+ 4,65 (s,3H); 7,85 (s,1H).

Beispiel 66

5-Chlormethyl-1-ethyl-3-methylthio-1.2.4-triazol

a) Eine Lösung von 91 g Acetaldehyd-Semicarbazon (Fp.134-136°C) in 700 ml Isopropanol werden bei 30°C portionsweise mit 30,4 g Natriumborhydrid versetzt. Nach gelinden Erwärmen setzt bei 60°C die Reaktion mit stürmischer Gasentwicklung ein. Man kühlt mit Eis und erhitzt das Reaktionsgemisch nach Abklingen der Reaktion noch 5 Stunden zum Rückfluß. Die Reaktionslösung wird mittels Eisessig auf einen pH-Wert von 6,0 gestellt. Die erhaltene Suspension wird über Nacht stehengelassen und abgesaugt. Nach Eindampfen des Filtrats, wird der Rückstand mit Wasser verrührt und abgesaugt. Es werden 35 g (= 38 % Ausbeute) der Verbindung der nachstehenden Formel

$$CH_3CH_2-NH-NH-CS-NH_2$$

in Form weißer Kristalle vom Fp. 137°C erhalten.

$^1$H-NMR-Daten (DMSO): 0,9 (t,3H); 2,62 (quint.,2H); 4,85 (t,1H); 7,0-7,6 (s-breit,2H); 8,6 (s,1H).

b) 35 g der unter a) erhaltenen Verbindung werden zusammen mit 17,5 g Natriummethylat und 25 g Glykolsäuremethylester in 250 ml Methanol 20 Stunden zum Sieden erhitzt.

Das Reaktionsgemisch wird mittels konz. Salzsäure auf einen pH-Wert von 4,0 gestellt und eingedampft. Der Rückstand wird mit siedenden Isopropanol mehrmals extrahiert. Der Isopropanol-Extrakt wird eingeengt, wobei das Produkt auskristallisiert. Nach Umkristallisation aus Wasser erhält man 1-Äthyl-5-hydroxymethyl-3-mercapto-1.2.4-triazol als Rohprodukt.

$^1$H-NMR-Daten ($CDCl_3$): 1,25 (t,3H); 4,0(q,2H); 4,5 (s,2H); 6,5-7,5 (s-breit,2H).

c) 20,4 g 1-Äthyl-5-hydroxymethyl-3-mercapto-1.2.4-triazol werden zusammen mit 7,17 g Natriummethylat in 170 ml Methanol gelöst und unter Kühlung mit 8,25 ml Methyljodid versetzt. Man rührt 2 Stunden bei Raumtemperatur und läßt das Gemisch über Nacht stehen. Nach Einstellen des pH-Wertes auf einen Wert von 7,0 wird das Reaktionsgemisch eingedampft und der Rückstand in Wasser aufgenommen. Man extrahiert dreimal mit Methylenchlorid, trocknet die organische Phase und engt sie zur Trockene ein. Man erhält 16,3 g (= 73,6 % Ausbeute) 1-Äthyl-5-hydroxymethyl-3-methylthio-1.2.4-triazol als gelbes Öl.

$^1$H-NMR-Daten ($CDCl_3$): 1,45(t,3H); 2,5 (s,3H); 4,2(q,2H); 4,7 (s,2H).

d) Eine Lösung von 16,3 g 1-Äthyl-5-hydroxymethyl-3-methyl-

thio-1.2.4-triazol in 38 ml Chloroform wird unter Eiskühlung unterhalb 25°C mit 39,8 ml Thionylchlorid versetzt. Man rührt 2 Stunden bei Raumtemperatur und anschließend 2,5 Stunden beim Erhitzen unter Rückfluß. Nach Stehenlassen über Nacht wird überschüssiges Thionylchlorid zusammen mit Chloroform im Vakuum abdestilliert, und nach Zugabe von Äther mittels 2n NaOH ein pH-Wert von 10,0 eingestellt. Die Ätherphase wird abgetrennt, die wäßrige Phase zweimal mit Äther extrahiert und die vereinigten organischen Extrakte eingedampft. Man erhält 15,73 g (= 87,4 %) der Titelverbindung als dunkles Öl.

$^1$H-NMR-Daten ($CDCl_3$): 1,5(t,3H); 2,55(s,3H); 4,17 (q,2H); 4,6(s,2H).

Beispiel 67

5-Chlormethyl-3-methylthio-1.2.4-triazol

Diese Verbindung wird analog den in Beispiel 66 beschriebenen Verfahrensweisen hergestellt, indem man zunächst Thiosemicarbazid mit Glyoxylsäuremethylester zu 5-Hydroxymethyl-3-mercapto-1.2.4-triazol (Fp. 177°C ) cyclisiert, diese Verbindung nachfolgend zu 5-Hydoxymethyl-3-methylthio-1.2.4-triazol (Fp. 113°C) methyliert und letztere Verbindung mittels Thionylchlorid zu 5-Chlormethyl-3-methylthio-1.2.4-triazol-hydrochlorid vom Fp.: 131°C umsetzt (s.a. Beispiel 64, Verfahrensweise b)).
Durch Zugabe von 2n NaOH wird die Titelverbindung in Form eines Öls freigesetzt.

Beispiel 68

1-Allyl-5-chlormethyl-1.2.4-triazol

Diese Verbindung wird analog den in Beispiel 64 und 65 beschriebenen Verfahrensweisen ausgehend von 1-Allyl-1.2.4-triazol über das Zwischenprodukt 1-Allyl-5-hydroxymethyl-1.2.4-triazol (Kp. 123°C/Torr) in Form eines Öls vom Kp. 222°C erhalten.

Beispiel 69

1 Benzyl-5-chlormethyl-1,2,4-triazol

Diese Verbindung wird analog den in Beispiel 64 und 65 beschriebenen Verfahrensweisen ausgehend von 1-Benzyl-1.2.4-triazol über 1-Benzyl-5-hydroxymethyl-1.2.4-triazol in Form eines Öls vom Kp. 293°C erhalten.

Patentansprüche:

1. 5-Halogenalkyl-1.2.4-triazole der Formel I, worin

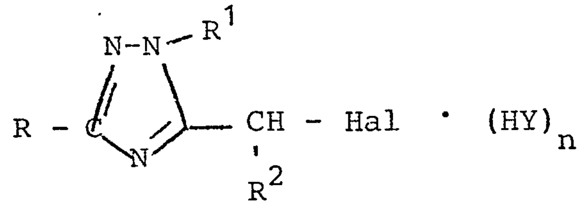 (I)

Hal = Chlor oder Brom,
Y = Halogen,
n = 0 oder 1
R = H, $(C_1-C_6)$-Alkyl, das durch Halogen $(C_1-C_4)$-Alkoxy oder $(C_1-C_4)$-Alkylthio substituiert sein kann, $(C_1-C_4)$-Alkylthio, Benzyl oder Phenylethyl, die im Phenylkern durch Halogen, Methoxy oder Methyl substituiert sein können,
$R^1$ = H, $(C_1-C_6)$-Alkyl, $(C_3-C_7)$-Cycloalkyl, Cyanethyl, Benzyl oder $(C_3-C_5)$-Alkenyl
$R^2$ = H oder Methyl bedeuten mit der Bedingung, daß im Falle R, $R^1$ = H, $R^2$ nicht Wasserstoff sein darf.

2. 5-Halogenalkyl-1.2.4-triazole der Formel I von Anspruch I, dadurch gekennzeichnet, daß
n = 0
Hal = Chlor oder Brom,
R = H, $(C_1-C_6)$-Alkyl, das durch Halogen, $(C_1-C_4)$-Alkoxy oder $(C_1-C_4)$-Alkylthio substituiert sein kann, Benzyl oder Phenylethyl, die im Phenylkern durch Halogen, Methoxy oder Methyl substituiert sein können,
$R^1$ = $(C_1-C_6)$-Alkyl, $(C_3-C_7)$-Cycloalkyl oder Cyanethyl und
$R^2$ = H oder Methyl bedeuten.

3. Verfahren zur Herstellung von Verbindungen der Formel I gemäß Ansprüchen 1 und 2, dadurch gekennzeichnet, daß man Amidrazone der Formel II

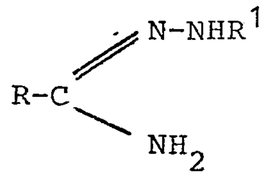

(II)

bzw. deren mineralsaure Salze mit α-Halogencarbonsäurehalogeniden bzw. -anhydriden der Formel III

$$R^2-CH(Hal)-\overset{O}{\overset{\|}{C}}-X \qquad III$$

umsetzt, die Zwischenprodukte dem Ringschluß mittels wasserabspaltender Mittel unterwirft und die erhaltenen Verbindungen der Formel I mit n = o ggf. in ihre Hydrohalogenide überführt.

4. Verfahren zur Herstellung von Verbindungen der Formel I mit R, $R^2$ = H und $R^1 \neq$ H, dadurch gekennzeichnet, daß man Verbindungen der Formel V, worin

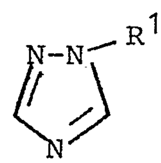

(V)

$R^1$ = $(C_1-C_6)$-Alkyl, $(C_3-C_7)$-Cycloalkyl, Cyanethyl, Benzyl oder $(C_3-C_5)$-Alkenyl bedeutet mit wäßrigem Formaldehyd zu einer Verbindung der Formel VI, worin

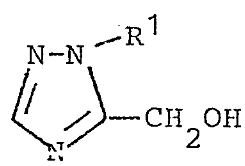

(VI)

$R^1$ die oben genannte Bedeutung besitzt, umsetzt, diese mit einem anorganischen Säurehalogenid reagieren läßt und das erhaltene Hydrohalogenid der Formel I (n = 1) ggf. mit einer Base umsetzt.

5. Verfahren zur Herstellung von Verbindungen der Formel I mit R = $(C_1-C_4)$-Alkylthio, $R^1$ = H, $(C_1-C_6)$-Alkyl oder $(C_3-C_7)$-Cycloalkyl und $R^2$ = H, dadurch gekennzeichnet, daß man Verbindungen der Formel VII, worin

$$R^1-NH-NH-\overset{S}{\overset{\|}{C}}-NH_2 \qquad (VII)$$

$R^1$ die obengenannte Bedeutung besitzt, mit einem Glykolsäurealkylester in Gegenwart von Alkalialkanolat zu Verbindungen der Formel VIII, worin

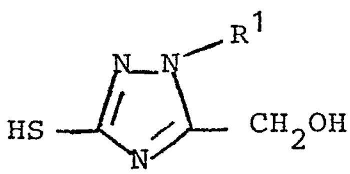 (VIII)

$R^1$ die obengenannte Bedeutung besitzt, umsetzt, diese mit einem Alkylhalognid zu einer Verbindung der Formel IX, worin $R^1$ die obengenannte Bedeutung

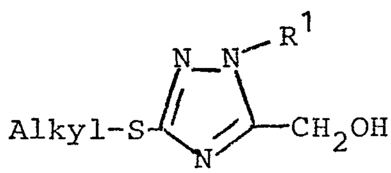 (IX)

besitzt und Alkyl einen $(C_1-C_4)$-Alkylrest bedeutet, alkyliert und letztere Verbindung mit einem anorganischen Säurehalogenid und ggf. anschließend mit einer Base umsetzt.

6. Verwendung von Verbindungen der Formel I gemäß Ansprüchen 1 und 2 zur Herstellung von Pflanzenschutzmitteln.